# EUROPEAN PATENT APPLICATION

(11) **EP 2 348 020 A1**
(43) Date of publication of application: **27.07.2011**
(21) Application number: 09382301.1
(22) Date of filing: 23.12.2009
(51) Int. Cl.: C07D 239/94

(54) **Preparation process of erlotinib**

(71) Applicant: Esteve Química, S.A., 08024 Barcelona (ES)
(72) Inventor: Batra Sanmartí, Martí, 08024, Barcelona (ES); Lupon Roses, Mª del Pilar, 08024, Barcelona (ES); Comely, Alexander Christian, Barcelona, 08028 (GB); Georges, Yohan Philippe, 08028, Barcelona (FR)
(74) Representative: ZBM Patents

(57) **Abstract**

Preparation process of erlotinib or its salts comprising: reacting 6,7-bis(2-methoxyethoxy)-4-methoxyquinazoline with 3-ethynylaniline or a salt thereof in the presence of a base and a reaction-inert solvent, and optionally, treating the compound obtained with a pharmaceutically acceptable acid to form the corresponding pharmaceutically acceptable salt. The compound 6,7-bis(2-methoxyethoxy)-4-methoxyquinazoline can be prepared either from 4-methoxyquinazoline-6,7-diol or 6,7-bis(2-methoxyethoxy)quinazolinone. The compounds 6,7-bis(2-methoxyethoxy)-4-methoxyquinazoline and 4-methoxyquinazoline-6,7-diol are new intermediates useful for the preparation of erlotinib or its salts.

## Description

The invention refers to a process for the preparation of an active pharmaceutical ingredient known as erlotinib, as well as some intermediates useful in this preparation process.

### BACKGROUND ART

Erlotinib is the International Non-proprietary Name (INN) of (N-(3-ethynylphenyl)-6,7-bis(2-methoxyethoxy)-4-quinazolinamine and CAS No. 183321-74-6. Erlotinib is administered in the form of the hydrochloride salt for the treatment of patients with locally advanced or metastatic non-small cell lung cancer after failure of at least one prior chemotherapy regimen. It is marketed under the trade name TARCEVA^{®} by OSI Pharmaceuticals.

The structure of erlotinib hydrochloride corresponds to the following formula:

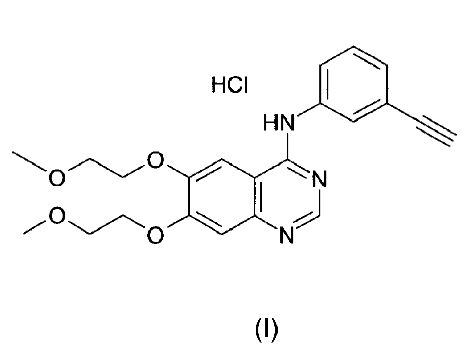

Different synthetic strategies for the preparation of erlotinib and its salts are known. Most of them go through a 4-substituted quinazoline intermediate having a suitable displaceable leaving group.

International patent application WO 96/30347 discloses 4-(substituted phenyl amino)quinazoline derivatives, processes for their preparation, pharmaceutical compositions containing them and its uses. According to this document, these compounds can be prepared by reacting 4-substituted quinazolines having a suitable displaceable leaving group with an appropriate amine or amine hydrochloride. Also according to this document the displaceable leaving groups can be one of the following groups: halo, aryloxy, alkysulfinyl, alkylsulfonyl such as trifluoromethanesulfonyloxy, arylsulfinyl, arylsulfonyl, siloxy, cyano, pyrazolo, triazolo, or tetrazolo. The preferred leaving group is the chloride and, in fact, is the only one exemplified. In particular, it is disclosed that erlotinib can be prepared by reaction of 3-ethynylaniline with 4-chloro-6,7-bis(2-methoxyethoxy)quinazoline in a mixture of pyridine and isopropanol. The free base is purified by chromatography on silica gel using a mixture of acetone and hexane. Then the free base is converted into the hydrochloride salt by treating a solution of erlotinib with hydrochloric acid in a mixture of chloroform and ether. The starting material 4-chloro-6,7-bis(2-methoxyethoxy)quinazoline is previously prepared from 6,7-bis(2-methoxyethoxy)-quinazolone by reaction with oxalylchoride in a solvent system containing chloroform and a small amount of N,N-dimethylformamide or by reaction with phosphoryl chloride in pyridine.

Other chlorinating agents to convert the 6,7-bis(2-methoxy-ethoxy)-quinazolone into 4-chloro-6,7-bis(2-methoxyethoxy)quinazoline have been described in the art. Thus, for instance, Venkateshappa Chandregowda et al., in Heterocycles 2007, vol. 71, pp. 39-48, carried out the conversion using thionyl chloride as solvent and N,N-dimethylformamide as catalyst. Petr Knesl et al., in Molecules 2006, vol. 11, pp. 286-297 carried out the conversion using phosphoryl chloride and N,N-diethylaniline. Both documents also describe the conversion of 4-chloro-6,7-bis(2-methoxyethoxy)quinazoline into erlotinib.

These processes have the drawback of going through the 4-chloroquinazoline intermediate which is isolated and which has been found to be unstable (cf. Venkateshappa Chandregowda et al., Organic Process Research & Development 2007, vol. 11, pp. 813-816; and Venkateshappa Chandregowda et al., in Synthetic Communications 2007, vol.37, pp. 3409-3415).

In addition, according to what is described in WO 2009/007984, the reproduction of the known processes described in the art -mentioned in the background of this international patent application - yielded relatively large amounts of impurities along with erlotinib. Among these impurities is the N-methoxyethyl impurity, namely N-[(3-ethynylphenyl)-2-methoxyethyl)]-6,7-bis(2-methoxyethoxy)-4-quinazolinamine. It is said that this impurity could not be eliminated by recrystallization but by column chromatography which cannot be used for large scale operations. This document describes a way to eliminate or reduce the impurity based on the isolation of erlotinib hydrochloride from a solvent medium comprising dimethyl sulfoxide and an alcoholic solvent.

Venkateshappa Chandregowda et al., in Synthetic Communications 2007, vol.37, pp. 3409-3415, also describe a process for preparing 6,7-dihydroxy-4-anilinoquinazoline derivatives which avoids the use of the unstable 4-chloroquinazoline intermediate. The process comprises converting the 6,7-bis(2-methoxyethoxy)-quinazolone into 6,7-bis-(2-methoxyethoxy)-quinazolin-4(3H)-thione by reacting the starting material with phosphorous pentasulfide and pyridine and maintaining the reaction mixture for 24 hours under reflux. The compound obtained is converted into 4-(methylthio)-6,7-bis-(2-methoxyethoxy)-quinazoline by dissolving the 6,7-bis-(2-methoxyethoxy)-quinazolin-4(3H)-thione in methanol in the presence of aqueous sodium hydroxide and methyl iodide. Finally, the compound obtained is converted into erlotinib hydrochloride by reacting it with 3-ethynylaniline and 3-ethynylaniline hydrochloride in isopropanol as solvent. The main drawback of this process is the use of sulphur-containing reactants. This type of reactants shows difficulties when operating at industrial scale due to their bad odours and in some cases their toxicity. Thus, bad odours are not only a problem when carrying out the reaction, but also in the residues generated such as the mother liquors. In addition, during the conversion of 4-(methylthio)-6,7-bis-(2-methoxyethoxy)-quinazoline into erlotinib, a mol of methanethiol is released for each mol of reacted 4-(methylthio)-6,7-bis-(2-methoxyethoxy)-quinazoline. The methanethiol is a colorless flammable gas with an extremely strong and repulsive smell and is highly toxic.

Therefore, a need still remains for an improved and commercially viable process of preparing erlotinib hydrochloride, suitable for large scale preparation, in terms of simplicity, chemical yields and purity of the final product.

### SUMMARY OF THE INVENTION

Inventors have found an improved process for the preparation of erlotinib or its salts based on the use of a new intermediate of formula (II) below.

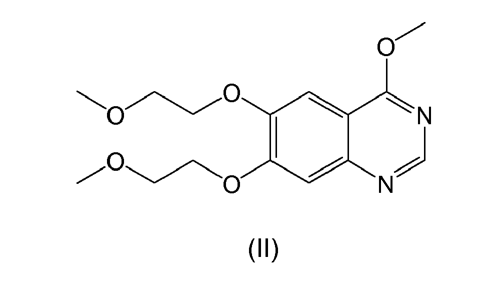

As it is illustrated in the Examples, the compound of formula (II) has the advantage that it is stable unlike the 4-chloroquinazoline intermediate used in the majority of the known processes. Its use is also advantageous compared to the 4-(methylthio)-6,7-bis-(2-methoxyethoxy)-quinazoline intermediate since the process of the present invention in which compound (II) intervenes, requires fewer reaction steps and avoids the use of sulphur-containing reactants, which show difficulties when operating at industrial scale due to their bad odours and in some cases their toxicity as has been explained above in the background art section.

The term "stable" as used herein, refers to a compound which possesses stability sufficient to allow manufacture and which maintains the integrity of the compound for a sufficient period of time to be useful for the purposes detailed herein (intermediate for use in the production of a therapeutic compound, isolable and storable), and which is stable in the reaction media of the reactions in which it intervenes.

Thus, an aspect of the present invention relates to a preparation process of erlotinib of formula (I) or its salts, 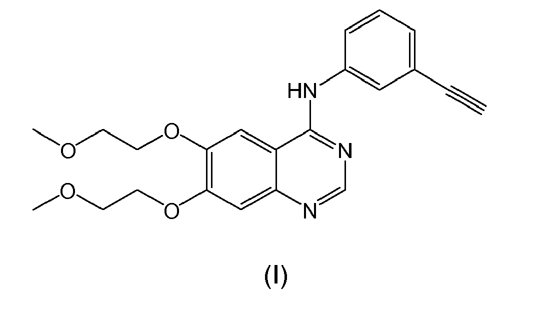 comprising: (a) reacting a compound of formula (II) with a compound of formula (III) or a salt thereof, in the presence of a base and a suitable solvent; and 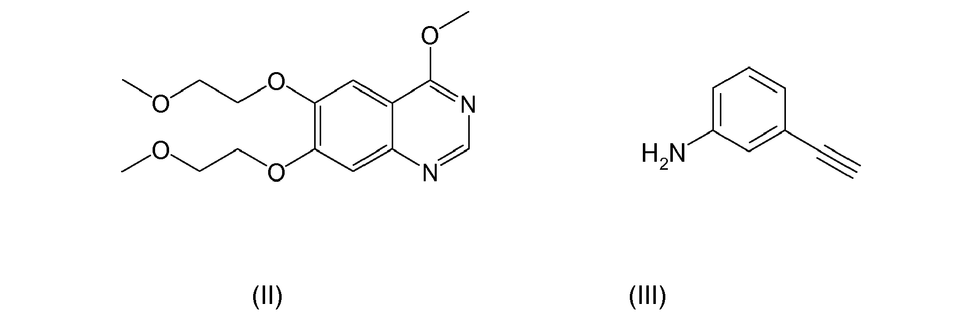 (b) optionally, treating the compound obtained in step (a) with a pharmaceutically acceptable acid to form the corresponding pharmaceutically acceptable salt.

Compound of formula (II) can be obtained from compound of formula (IV) or from compound of formula (VII) by the methods explained in detail below. 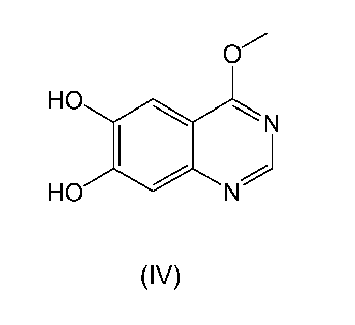 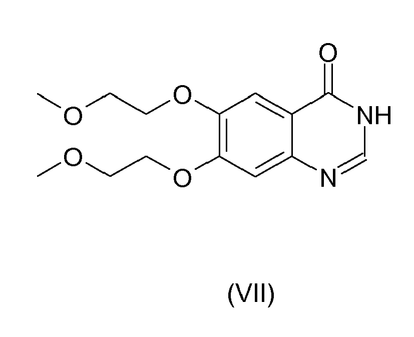 Compounds of formula (II) and (IV) or their salts are new and form part of the invention.

These compounds allow the preparation of erlotinib or its salts by a simple process which proceeds with high yields and purity. Therefore, also part of the invention is the use of the compound of formula (II) or of the compound of formula (IV) as intermediates for the preparation of erlotinib or its pharmaceutically acceptable salts.

### DETAILED DESCRIPTION OF THE INVENTION

As mentioned above, erlotinib or its salts can be prepared by a process comprising (a) reacting a compound of formula (II) with a compound of formula (III) or a salt thereof, in the presence of a base and a suitable solvent; and (b) optionally, treating the compound obtained in step (a) with a pharmaceutically acceptable acid to form the corresponding pharmaceutically acceptable salt.

The compound of formula (III) can be used in its free form or as a salt thereof. Preferably, the salt of the compound of formula (III) is the hydrochloride salt.

In a preferred embodiment, the base is selected from *n*-butyllithium, *sec*-butyl lithium, hexyllithium, lithium diisopropylamide, *tert*-butyllithium, lithium hexamethyldisilazide, R₂NLi, and RLi, where R is a (C₁-C₈)-alkyl. In a more preferred embodiment, the base is selected from: *n*-butyllithium, *sec-*butyllithium, hexyllithium and lithium diisopropylamide (LDA). In an even more preferred embodiment, the base is *sec*-butyllithium or LDA.

Preferably, the molar ratio of 3-ethynylaniline:base relative to compound (II) is comprised between 1.1 : 2.2 and 1.4 : 3. More preferably, the molar ratio is comprised between 1.2 : 2.4 and 1.4 : 2.9. Even more preferably, the molar ratio is 1.4 : 2.9.

The reaction is carried out in a wide range of temperatures. Preferably, the reaction is carried out at a temperature comprised between -30°C and 50°C. more preferably, the reaction is carried out at a temperature comprised between -15 °C and room temperature. Preferably, the reaction is carried out at room temperature. By room temperature it is understood a temperature comprised between 20 and 30 °C. Thus, unlike most of the prior art process, the process of the present invention can be carried out without heating, which is advantageous since it is easy to carry out at industrial scale.

Generally, the reaction is carried out in the presence of a reaction-inert solvent. Preferably, the solvent is selected from (C₄-C₁₀)-alkyl or cycloalkyl ethers such as tetrahydrofuran, dioxane or methyl *tert*-butyl ether, (C₆-C₈)-alkanes or cycloalkanes such as hexane, heptane or cyclohexane, and (C₆-C₈)-aromatic hydrocarbons such as toluene or xylene. More preferably the solvent is a (C₄-C₁₀)-alkyl or cycloalkyl ether.

The expression reaction-inert solvent in the context of the present invention refers to a solvent which does not interact with the starting materials, reagents, intermediates or products in a manner which adversely affects the yield of the desired product.

Erlotinib can be isolated directly from the reaction medium as a base or as a salt as it is illustrated in the Examples.

The pharmaceutically acceptable salts of the compound of formula (I) include acid addition salts such as the hydrochloride, hydrobromide, hydrofluoride, hydroiodide, sulphate or bisulphate, phosphate, hydrogen phosphate, acetate, besylate, citrate, fumarate, gluconate, lactate, maleate, mesylate, succinate or tartrate salts. Preferably, the salt of the compound of formula (I) is the hydrochloride salt.

These salts can be prepared conventionally, for instance, by mixing a solution of the free base with the corresponding acid in a suitable solvent system, eg. ethanol or tetrahydrofuran, and recovering the acid addition salt either as precipitate or by evaporation of the solvent from the solution. The solvent system can be either aqueous, non-aqueous or partially aqueous. Usually, the salts are prepared by contacting stoichiometric amounts of the acid and basic entities. Erlotinib or its salts can exist in solvated, as well as unsolvated forms, including hydrated forms, i.e. erlotinib can contain in its structure stoichiometric amounts of solvent in the case of solvates, or of water in the case of hydrates. It is to be understood that the invention encompasses all such solvated, as well as unsolvated, forms. The obtention of solvates and hydrates depends on the solvent or mixture of solvents used and the crystallization conditions that can be determined by the skilled person.

Compound of formula (II) can be prepared by reacting a compound of formula (IV), with a compound of formula (V).

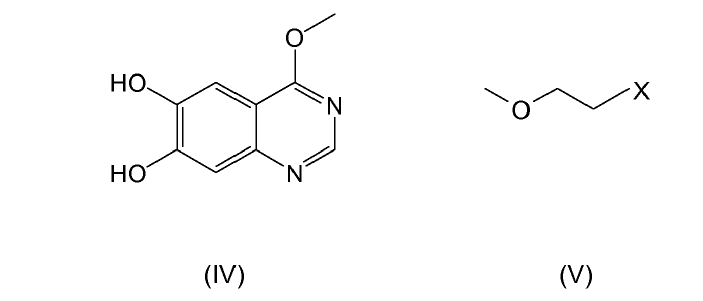

In the previous formula X is a leaving group. Examples of appropriate leaving groups are a halogen such as chloride, bromide or iodide, or a sulphonic ester of formula OSO₂R₁, where R₁ is a radical selected from (C₁-C₄)-alkyl, phenyl, and phenyl mono- or disubstituted by a (C₁-C₄)-alkyl radical. In a preferred embodiment the leaving group is a bromide, a mesylate (OSO₂Me), a besylate (OSO₂Ph) or a tosylate (OSO₂PhMe). In a more preferred embodiment, the leaving group is bromide. When the leaving group is chloride, generally the reaction between compound (IV) and (V) is carried out in the presence of a small amount of sodium iodide.

Compund of formula (II) can be in form of an acid salt such as those mentioned for compound (I). They can be prepared by conventional methods such as mixing a solution of the free base with the corresponding acid in a suitable solvent system and recovering the acid addition salt either as precipitate or by evaporation of the solvent from the solution, as it has been explained for compound (I). Examples of suitable solvents are (C₁-C₄)-alcohols.

The compound of formula (IV) can be in the form of its neutral catechol/free base or in form of an acid salt, for instance hydrochloride or in form of a salt of a metal such as the sodium or disodium salt. In the case that the starting material (IV) is in the form of a salt of an acid, the reaction is carried out in the presence of extra base, as appropriate, in order to convert the salt into the neutral catechol/free base.

These salts can be prepared conventionally, for instance, acid salts of compound of formula (IV) can be obtained by mixing a solution of the free base with the corresponding acid in a suitable solvent system. Basic salts can be prepared by mixing a solution of the free base with the corresponding base, for instance sodium methoxide or sodium hydroxide in a suitable solvent system. Examples of suitable solvents are (C₁-C₄)-alcohols. The salts can be recovered either as precipitate or by evaporation of the solvent from the solution.

The reaction between the compound of formula (IV) either in the form of its neutral catechol/free base or in salt form with the compound of formula (V) is generally carried out in the presence of an inorganic or organic base. Examples of appropriate bases include alkaline metal carbonates such as potassium carbonate or sodium carbonate, alkaline metal hydroxides such as potassium hydroxide or sodium hydroxide, alkaline hydrides such as sodium hydride or lithium hydride, RLi, or R₂NLi where R is a (C₁-C₈)-alkyl.

Preferably, the molar ratio of compound (IV)/compound (V) is comprised between 1 : 2 to 1 : 6. More preferably, the molar ratio is 1 : 4.

Generally, the reaction is carried out in the presence of a reaction-inert solvent. Preferably, the solvent is selected from (C₄-C₁₀)-alkyl or cycloalkyl ethers such as tetrahydrofuran, (C₆-C₈)-alkanes or cycloalkanes such as hexane, heptane or cyclohexane, (C₆-C₈)-aromatic hydrocarbons such as toluene or xylene, (C₃-C₈)-ketones such as acetone, methylethylketone or methylisobutylketone, N,N-dimethylformamide (DMF) and N-methyl pyrrolidone (NMP).

Generally, the reaction is carried out at a temperature comprised between 0 °C and 40 °C. Preferably, it is carried out at a temperature about 30 °C.

The compound of formula (IV) can be prepared by reacting in one-pot a compound of formula (VI) with a halogenating agent to yield 4-halo-6,7-dihydroxyquinazoline, and then with methanol to yield the compound of formula (IV).

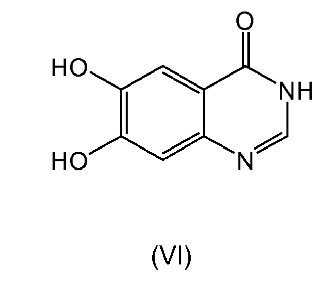

Examples of appropriate halogenating agents are SOCl₂, (COCl)₂, PCl₃, PCl₅, PBr₃, PBr₅ or POCl₃ which yield either 4-chloro-6,7-dihydroxyquinazoline or 4-bromo-6,7-dihydroxyquinazoline, these being intermediates not isolated from the reaction medium.

Preferably the 4-halo dihydroxyquinazoline is 4-chloro-6,7 dihydroxyquinazoline and the halogenating agent is a chlorinating agent. In a preferred embodiment the halogenating agent is POCl₃

The reaction can be carried out without solvent or in the presence of a reaction-inert solvent. Examples of appropriate solvents include (C₄-C₁₀)-alkyl or cycloalkyl ethers such as tetrahydrofuran, (C₆-C₈)-alkanes or cycloalkanes such as hexane, heptane or cyclohexane, (C₆-C₈)-aromatic hydrocarbons such as toluene or xylene, or N,N-dimethylformamide. More preferably, the halogenation reaction is carried out using N,N-dimethylformamide as solvent.

Especially good yields are obtained when POCl₃ is used as chlorinating agent and DMF is used as solvent of the chlorination. POCl₃ is a preferred halogenating agent to be used at industrial scale since it is less dangerous cheap, widely used and does not generate such gases as SO₂, CO or CO₂ as do SOCl₂ and (COCl)₂, respectively.

Generally, this reaction is carried out at a temperature comprised between 80 °C and 150 °C. In a particular embodiment, the reaction is carried out at about 120 °C.

These process steps are advantageous since they can be carried out in one pot without isolating the 4-halo-6,7-dihydroxyquinazoline intermediates.

Alternatively, the compound of formula (II) can be prepared in one-pot by reacting a compound of formula (VII) with a halogenating agent to yield a 4-halo-6,7-bis-(2-methoxyethoxy)-quinazoline, and then with methanol yielding the compound of formula (II).

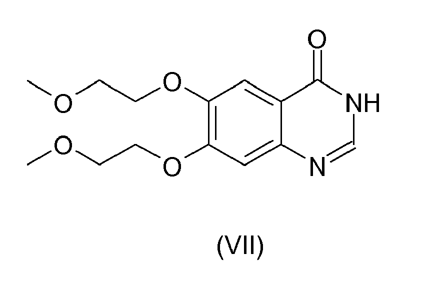

Examples of appropriate halogenating agents are SOCl₂, (COCl)₂, PBr₃, PBr₅, PCl₃, PCl₅, and POCl₃ which yield either 4-chloro-6,7-bis-(2-methoxyethoxy)-quinazoline or 4-bromo-6,7-bis-(2-methoxyethoxy)-quinazoline, this intermediate not being isolated from the reaction medium.

Preferably, the 4-halo-6,7-bis-(2-methoxyethoxy)-quinazoline is 4-chloro-6,7-bis-(2-methoxyethoxy)-quinazoline and the halogenating agent is a chlorinating agent. In a preferred embodiment the halogenating agent is POCl₃

Preferably, the halogenation reaction is carried out using N,N-dimethylformamide. Especially good yields are obtained when POCl₃ is used as chlorinating agent and DMF is used as solvent of the chlorination. Other reaction-inert solvents solvents such (C₄-C₁₀)alkyl or cycloalkyl ethers such as tetrahydrofuran, (C₆-C₈)alkanes or cycloalkanes such as hexane, heptane or cyclohexane, (C₆-C₈) aromatic hydrocarbons such as toluene or xylene can also be used.
6,7-bis(2-methoxy-ethoxy)-quinazolone can be prepared by known methods in the art. Its preparation is described, for instance, in Preparation 1 of WO 96/30347. 6,7-dimethoxy-4-(3H)-quinazoline is commercially available.

Finally, as mentioned above, the compounds of formula (II) and (IV) or their salts are new and form part of the invention. Also forming part of the invention is the use of these compounds as intermediates for the preparation of erlotinib or its salts.

Throughout the description and claims the word "comprise" and variations of the word are not intended to exclude other technical features, additives, components, or steps.

Additional objects, advantages and features of the invention will become apparent to those skilled in the art upon examination of the description or may be learned by practice of the invention. The following examples and drawings are provided by way of illustration and they are not intended to be limiting of the present invention. Furthermore, the present invention covers all possible combinations of particular and preferred embodiments described herein.

### EXAMPLES

The purity of the erlotinib/erlotinib hydrochloride of the examples has been determined by HPLC. HPLC conditions: Column: XBridge C₁₈; eluent: NH₄HCO₃ pH 8/ACN (95:5-7 min-0:100); sample: 1 mg in 2 ml methanol; detection: 254nm.

### Example 1: Preparation of 6,7-dihydroxyauinazolinone

In a 250 mL flask, a mixture of 6,7-dimethoxy-4-(3H)-quinazoline (20.00 g, 97.0 mmol) and 48% HBr (200 g, 133 mL) was heated at 110 °C and was stirred for 1 h. The mixture was heated to 140 °C and was stirred for 15 h. The resulting suspension was cooled to 20 °C and was filtered. The collected solid was suspended in H₂O (150 mL) and NH₄OH was added to pH 9. The suspension was filtered and the resulting solid was suspended in 1 M NaHCO₃ (150 mL). The solid was filtered, was washed with H₂O (5 mL) and was dried to give the title compound as a white solid (16.82 g, 97% yield). ¹H NMR (d6-DMSO, ppm): δ 7.79 (s, 1 H), 7.32 (s, 1 H), 6.88 (s, 1 H).

### Example 2: Preparation of 4-methoxyguinazoline-6,7-diol

In a 250 mL flask, a mixture of 6,7-dihydroxyquinazolinone (15.00 g, 84.2 mmol) and DMF (1.70 mL, 21.9 mmol, 0.26 eq.) in POCl₃ (38.5 mL, 421 mmol, 5 eq.) was stirred at 120 °C for 3 h or until total dissolution of the solid is observed. POCl₃ was distilled under vacuum and to the resulting foam were added toluene (50.0 mL) and K₂CO₃ (11.63 g, 84.2 mmol, 1 eq.). The mixture was cooled to 0 °C and methanol (70.0 mL) was added carefully slowly keeping the temperature under 40 °C. The resulting suspension was stirred at room temperature for 45 min and the solvents were removed under vacuum to provide a solid foam. H₂O (150 mL) was added slowly and 50% NaOH (18 mL) was added at 0 °C to pH 5. The resulting solid was filtered and was washed with H₂O (20 mL) to give the title compound as a yellow solid (12.81 g, 79% yield). ¹H NMR (d⁶-DMSO, ppm): δ 8.47 (s, 1 H), 7.27 (s, 1 H), 7.11 (s, 1H), 4.02(s,3H).

### Example 3: Preparation of 6,7-bis(2-methoxyethoxy)-4-methoxyquinazoline

In a 50 mL flask, a mixture of 4-methoxyquinazoline-6,7-diol (4.29 g, 22.3 mmol), K₂CO₃ (15.42 g, 111.6 mmol, 5 eq.) and bromomethyl ethyl ether (8.37 mL, 89.2 mmol, 4 eq.) in anhydrous DMF (25 mL) was stirred at 30 °C for 24 h. H₂O (30 mL) was added and the aqueous layer was extracted with toluene (3 x 20 mL). The combined organic layers were dried over MgSO₄ and were evaporated under reduced pressure to give the title compound as an off-white solid (5.52 g, 80% yield). ¹H NMR (d⁶-DMSO, ppm): δ 8.67 (s, 1 H), 7.40 (s, 1 H), 7.25 (s, 1 H), 4.31-4.26 (m, 4H), 4.15 (s, 3H), 3.88-3.85 (m, 4H), 3.49 (s, 3H), 3.49 (s, 3H).

### Example 4: Preparation of erlotinib from 6,7-bis(2-methoxvethoxy)-4-methoxyquinazoline using n-BuLi

In a 100 mL flask, a solution of 3-ethynylaniline (1.46 mL, 12.97 mmol, 2 eq.) in anhydrous THF (10 mL) was cooled to 0 °C under N₂. n-BuLi (2.35 M in toluene, 11.04 mL, 25.9 mmol, 4 eq.) was added slowly at 0 °C and the mixture was stirred at 0 °C for 30 min. A solution of 6,7-bis(2-methoxyethoxy)-4-methoxyquinazoline (2.00 g, 6.48 mmol) in anhydrous tetrahydrofuran (THF) (15 mL) was added over 10 min and the reaction mixture was stirred at room temperature for 4 h. The reaction was quenched with H₂O (5 mL). THF was removed under reduced pressure and H₂O (20 mL) was added. The aqueous layer was extracted with AcOEt (3 x 20 mL). The combined organic layers were dried over MgSO₄ and were evaporated under reduced pressure to give a brown oil (3.11 g). The crude was purified by flash chromatography on silica gel (eluent: CH₂Cl₂ to CH₂Cl₂/MeOH 95/5) to give erlotinib as a yellow solid (1.78 g, 70%). ¹H NMR (CDCl₃, ppm): δ 8.66 (s, 1 H), 7.86 (s, 1 H), 7.76 (m, 1 H), 7.35 (m, 1 H), 7.28 (s, 1 H), 7.22 (s, 1 H), 7.21 (s, 1 H), 4.36-4.25 (m, 4H), 3.85-3.81 (m, 4H), 3.48 (s, 3H), 3.46 (s, 3H), 3.10 (s, 1 H).

### Example 5: Preparation of erlotinib hydrochloride from 6,7-bis(2-methoxvethoxy)-4-methoxyguinazoline using n-BuLi

In a 100 mL flask, a solution of 3-ethynylaniline (511 µL, 4.54 mmol, 1.4 eq.) in anhydrous THF (5 mL) was cooled to 0 °C under N₂. n-BuLi (2.35 M in toluene, 4.00 mL, 9.40 mmol, 2.9 eq.) was added slowly at 0 °C and the mixture was stirred at 0 °C for 30 min. A solution of 6,7-bis(2-methoxyethoxy)-4-methoxyquinazoline (1.00 g, 3.24 mmol) in anhydrous THF (7.5 mL) was added slowly and the reaction mixture was stirred at room temperature for 1 h. The reaction was quenched with H₂O (2 mL). THF was removed under reduced pressure and H₂O (10 mL) was added. The aqueous layer was extracted with ethyl acetate (3 x 10 mL). The combined organic layers were dried over MgSO₄ and were evaporated under reduced pressure to give erlotinib as a brown paste (1.43 g, >90% conversion). The paste was dissolved in THF (10 mL), 2 M HCl (2 mL) was added and the mixture was stirred for 30 min at 0 °C. The resulting suspension was filtered and was washed with THF (3 mL) to give the title compound as an off-white solid (855 mg, 61 % yield, 98.8% HPLC). ¹H NMR (d⁶-DMSO, ppm): δ 11.51 (s, 1 H), 8.83 (s, 1 H), 8.42 (s, 1 H), 7.86 (s, 1 H), 7.77 (m, 1 H), 7.48 (m, 1 H), 7.41-7.37 (m, 2H), 4.40-4.35 (m, 4H), 4.33-4.29 (m, 4H), 4.26 (s, 3H), 3.34 (s, 6H).

### Example 6: Preparation of erlotinib hydrochloride from 6,7-bis(2-methoxvethoxy)-4-methoxyguinazoline using n-BuLi

In a 50 mL flask, a solution of 3-ethynylaniline (511 µL, 4.54 mmol, 1.4 eq.) in anhydrous THF (10 mL) was cooled to 0 °C under N₂. n-BuLi (2.5 M in toluene, 3.76 mL, 9.40 mmol, 2.9 eq.) was added slowly at 0 °C and the mixture was stirred at 0 °C for 30 min. A solution of 6,7-bis(2-methoxyethoxy)-4-methoxyquinazoline (1.00 g, 3.24 mmol) in anhydrous THF (7.5 mL) was added slowly and the reaction mixture was stirred at room temperature for 15 h (100% conversion, 89.0% HPLC). The reaction was quenched with H₂O (5 mL) and 6 M HCl (2.0 mL) was added slowly until the precipitation of a solid began. Further 6 M HCl (0.6 mL, 3.6 mmol, 1.1 eq.) was added and the mixture was stirred for 3 h at RT. The resulting solid was filtered and was washed with THF (3 mL) to give the title compound as an off-white solid (1.214 g, 87% yield, 98.5% HPLC).

### Example 7: Preparation of erlotinib hydrochloride from 6,7-bis(2-methoxvethoxy)-4-methoxyguinazoline using LDA

In a 50 mL flask, a solution of 3-ethynylaniline (511 µL, 4.54 mmol, 1.4 eq.) in anhydrous THF (10 mL) was cooled to 0 °C under N₂. LDA (1.49 M in THF/heptane/ethylbenzene, 6.26 mL, 9.40 mmol, 2.9 eq.) was added slowly at 0 °C and the mixture was stirred at 0 °C for 30 min. A solution of 6,7-bis(2-methoxyethoxy)-4-methoxyquinazoline (1.00 g, 3.24 mmol) in anhydrous THF (7.5 mL) was added slowly and the reaction mixture was stirred at room temperature for 15 h (100% conversion, 88.8% HPLC). The reaction was quenched with H₂O (5 mL) and 6 M HCl (3.6 mL) was added slowly until the precipitation of a solid began. Further 6 M HCl (0.6 mL, 3.6 mmol, 1.1 eq.) was added and the mixture was stirred for 3 h at room temperature. The resulting solid was filtered and was washed with THF (3 mL) to give the title compound as an orange solid (1.353 g, 97% yield, 98.0% HPLC).

### Example 8: Preparation of erlotinib hydrochloride from 6,7-bis(2-methoxvethoxy)-4-methoxyguinazoline using sec-BuLi

In a 50 mL flask, a solution of 3-ethynylaniline (511 µL, 4.54 mmol, 1.4 eq.) in anhydrous THF (10 mL) was cooled to 0 °C under N₂. sec-BuLi (1.3 M in cyclohexane, 7.23 mL, 9.40 mmol, 2.9 eq.) was added slowly at 0 °C and the mixture was stirred at 0 °C for 30 min. A solution of 6,7-bis(2-methoxyethoxy)-4-methoxyquinazoline (1.00 g, 3.24 mmol) in anhydrous THF (7.5 mL) was added slowly and the reaction mixture was stirred at room temperature for 15 h (100% conversion, 95.4% HPLC). The resulting suspension was quenched with H₂O (5 mL) and 6 M HCl (2.0 mL) was added slowly until the suspension dissolved and a new precipitation began. Further 6 M HCl (0.6 mL, 3.6 mmol, 1.1 eq.) was added and the mixture was stirred for 3 h at room temperature. The resulting solid was filtered and was washed with THF (3 mL) to give the title compound as a white solid (1.272 g, 91 % yield, 99.0% HPLC).

### Example 9: Preparation of erlotinib from 6,7-bis(2-methoxyethoxy)-4-methoxyquinazoline using sec-BuLi

In a 50 mL flask, a solution of 3-ethynylaniline (511 µL, 4.54 mmol, 1.4 eq.) in anhydrous THF (10 mL) was cooled to 0 °C under N₂. sec-BuLi (1.3 M in cyclohexane, 7.23 mL, 9.40 mmol, 2.9 eq.) was added slowly at 0 °C and the mixture was stirred at 0 °C for 30 min. A solution of 6,7-bis(2-methoxyethoxy)-4-methoxyquinazoline (1.00 g, 3.24 mmol) in anhydrous THF (7.5 mL) was added slowly and the reaction mixture was stirred at room temperature for 15 h (100% conversion, 94.3% HPLC). The resulting suspension was quenched with H₂O (5 mL) and the mixture was stirred for 5 h. The resulting solid was filtered and was washed with THF (3 mL) to give the title compound as a white solid (0.808 g, 63% yield, 99.0% HPLC).

### Example 10: Preparation of erlotinib hydrochloride from 6,7-bis(2-methoxyethoxy)-4-methoxyauinazoline using sec-BuLi and 3-ethynylaniline hydrochloride

In a 50 mL flask, a solution of 3-ethynylaniline hydrochloride (697 mg, 4.54 mmol, 1.4 eq.) in anhydrous THF (10 mL) was cooled to 0 °C under N₂. *sec-*BuLi (1.3 M in cyclohexane, 10.72 mL, 13.95 mmol, 4.3 eq.) was added slowly at 0 °C and the mixture was stirred at 0 °C for 30 min. A solution of 6,7-bis(2-methoxyethoxy)-4-methoxyquinazoline (1.00 g, 3.24 mmol) in anhydrous THF (7.5 mL) was added slowly and the reaction mixture was stirred at room temperature for 15 h (100% conversion, 95.5% HPLC). The resulting suspension was quenched with H₂O (5 mL) and 6 M HCl (1.2 mL) was added slowly until the suspension dissolved and a new precipitation began. Further 6 M HCl (0.6 mL, 3.6 mmol, 1.1 eq.) was added and the mixture was stirred for 1 h at room temperature. The resulting solid was filtered and was washed with THF (3 mL) to give the title compound as an off-white solid (1.40 g, 100% yield, 97.1 % HPLC).

### Example 11: Preparation of erlotinib hydrochloride from 6,7-bis(2-methoxyethoxy)-4-methoxyguinazoline using HexLi

In a 50 mL flask, a solution of 3-ethynylaniline (511 µL, 4.54 mmol, 1.4 eq.) in anhydrous THF (10 mL) was cooled to 0 °C under N₂. HexLi (2.2 M in hexane, 4.27 mL, 9.40 mmol, 2.9 eq.) was added slowly at 0 °C and the mixture was stirred at 0 °C for 30 min. A solution of 6,7-bis(2-methoxyethoxy)-4-methoxyquinazoline (1.00 g, 3.24 mmol) in anhydrous THF (7.5 mL) was added slowly and the reaction mixture was stirred at RT for 15 h (100% conversion, 90.0% HPLC). The reaction was quenched with H₂O (5 mL) and 6 M HCl (2.5 mL) was added slowly until the precipitation of a solid began. Further 6 M HCl (0.6 mL, 3.6 mmol, 1.1 eq.) was added and the mixture was stirred for 3 h at RT. The resulting solid was filtered and was washed with THF (3 mL) to give the title compound as a yellow solid (1.097 g, 79% yield, 97.5% HPLC).

### Example 12: Preparation of 6,7-bis(2-methoxyethoxy)-4-methoxyquinazoline from 6,7-bis(2-methoxyethoxy)quinazolinone

In a 25 mL flask, a mixture of 6,7-bis(2-methoxyethoxy)quinazolinone (1.00 g, 3.40 mmol) and DMF (68 µL, 0.88 mmol, 0.26 eq.) in POCl₃ (1.55 mL, 17.0 mmol, 5 eq.) was stirred at 120 °C for 30 min. POCl₃ was distilled under vacuum and, to the resulting foam, toluene (3.4 mL) and K₂CO₃ (470 mg, 3.40 mmol, 1 eq.) were added. The mixture was cooled to 0 °C and MeOH (4.8 mL) was added slowly keeping the temperature below 40 °C. The resulting suspension was stirred at room temperature for 30 min and the solvents were removed under vacuum. H₂O (15 mL) was added slowly and 1 M NaOH was added at 0 °C to pH 6. The aqueous layer was extracted with toluene (3 x 20 mL). The combined organic layers were dried over MgSO₄ and were evaporated under reduced pressure to give the title compound as a yellow solid (930 mg, 89% yield).

### Example 13: Preparation of 6,7-bis(2-methoxyethoxy)-4-methoxyguinazoline from 6,7-bis(2-methoxyethoxy)quinazolinone

In a 100 mL flask, a mixture of 6,7-bis(2-methoxyethoxy)quinazolinone (20.00 g, 67.9 mmol) and DMF (1.37 mL, 17.7 mmol, 0.26 eq.) in POCl₃ (31.1 mL, 339.8 mmol, 5 eq.) was stirred at 80 °C for 30 min. POCl₃ was distilled under vacuum and, to the resulting foam, toluene (68 mL) and K₂CO₃ (9.40 g, 67.9 mmol, 1 eq.) were added. The mixture was cooled to 0 °C and MeOH (96 mL) was added slowly keeping the temperature below 40 °C. The resulting suspension was stirred at room temperature for 30 min and the solvents were removed under vacuum. H₂O (100 mL) was added slowly and 1 M NaOH was added at 0 °C to pH 6. The aqueous layer was extracted with toluene (4 x 100 mL). The combined organic layers were washed with brine (10 mL), were dried over MgSO₄ and were evaporated under reduced pressure to give the title compound as an off-white solid (19.69 g, 94% yield).

### Comparative Example 14: Reproduction of the process described in Heterocycles, 2007, vol.71, pp. 39-48 for the preparation of erlotinib hydrochloride from 4-chloro-6,7-bis(2-methoxyethoxy)quinazoline

In a 50 mL flask, 3-ethynylaniline (360 µL, 3.20 mmol, 1 eq.) was added to a solution of 4-chloro-6,7-bis(2-methoxyethoxy)quinazoline (1.00 g, 3.20 mmol, prepared according to Heterocycles, 2007, vol.71, pp. 39-48, namely from henceforth compound A) in anhydrous DMF (10 mL). The mixture was stirred at 80 °C for 1 h. The mixture was cooled to 10 °C and the resulting solid was filtered and was washed with cold DMF to give the title compound as a white solid (1.238 g, 90% yield, 100.0% HPLC).

### Comparative Example 15: Reproduction of the process described in Synthetic Communications, 2007, vol.37 , pp. 3409-3415 for the preparation of erlotinib hydrochloride from 4-(methylthio)-6,7-bis-(2-methoxyethoxy) quinazoline

In a 50 mL flask, 3-ethynylaniline (410 µL, 3.64 mmol, 1.18 eq.) and 3-ethynylaniline hydrochloride (559 mg, 3.64 mmol, 1.18 eq.) were added to a solution of 4-(methylthio)-6,7-bis-(2-methoxyethoxy)quinazoline (1.00 g, 3.08 mmol, prepared according to Synthetic Communications, 2007, vol. 37, pp.3409-3415, namely from henceforth compound B) in anhydrous isopropanol (IPA) (11 mL). The mixture was refluxed for 15 h. The mixture was cooled to 10 °C and the resulting solid was filtered and was washed with cold IPA to give the title compound as a white solid (1.098 g, 83% yield, 93.4% HPLC, impurity i₁ 4.1%, impurity i₂: 1.2%).

### Comparative Example 16: Reproduction of the process described in US5747498 for the preparation of erlotinib hydrochloride from 4-chloro-6,7-bis(2-methoxyethoxy)quinazoline

In a 50 mL flask, 3-ethynylaniline (421 µL, 3.74 mmol, 1.1 eq.) and anhydrous pyridine (302 µL, 3.74 mmol, 1.1 eq.) were added to a solution of 4-chloro-6,7-bis(2-methoxyethoxy)quinazoline (1.06 g, 3.40 mmol, prepared according to US 5747498) in anhydrous IPA (15 mL). The mixture was stirred at 80 °C for 1 h. The solvents were removed under vacuum and the residue was suspended in a mixture of CH₂Cl₂ (18 mL) and MeOH (2 mL). The organic layer was washed with 1 M NaHCO₃ (10 mL), was dried over MgSO₄ and was evaporated under reduced pressure to give erlotinib as a yellow solid (1.234 g, 92% conversion, 87.6% HPLC). The solid was dissolved in a minimum of CHCl₃ (2.4 mL, 2 mL/g) and Et₂O (7.4 mL, 6 mL/g) was added. HCl (4 M solution in dioxane, 0.85 mL, 3.36 mmol, 1.1 eq.) was added slowly and the mixture was stirred at RT for 1 h. The resulting solid was filtered and was washed with Et₂O (2 mL) to give the title compound as a white solid (1.186 g, 81 % yield from 6,7-bis(2-methoxyethoxy)quinazolinone, 82.4% HPLC, impurity iₐ: 11.4% (hydrolisis product of 4-chloro-6,7-bis(2-methoxyethoxy)quinazoline, *i.e.* 6,7-bis(2-methoxyethoxy)quinazolinone), impurity i_{b}: 4.4%, impurity i_{c}:1.9%).

Purity and yield data for the erlotinib hydrochloride obtained in the previous Examples and comparative Examples are provided in Table 1.

**Table 1:**

| Example | Conversion 4h % | Conversion 15 % | Purity of the crude % | Yield after Yield after precipitation % | Purity % |
|---|---|---|---|---|---|
| Preparation of erlotinib hydrochloride | | | | | |
| Example 6 | 95.5 | 100 | 89.0 | 87 | 98.5 |
| Example 8 | 95.2 | 100 | 95.4 | 91 | 99.0 |
| Example 11 | - | 100 | 90.0 | 79 | 97.5 |
| Example 7 | 89.0 | 100 | 88.8 | 97 | 98.0 |
| Example 10 | 100 | 100 | 95.5 | 100 | 97.1 |
| Comparativ e Example 14 | - | - | - | 90 | 100 |
| Comparativ e Example 15 | - | - | - | 83 | 93.4 |
| Comparativ e Example 16 | - | 92 | 87.6 | 81 | 82.4 |
| Preparation of erlotinib | | | | | |
| Example 4 | - | - | - | 70 | - |
| Example 9 | 95.2 | 100 | 94.3 | 63 | 99.0 |

### Comparative Example 17: Stability tests

The stability of the compound 6,7-bis(2-methoxyethoxy)-4-methoxyquinazoline (Example 13) has been compared with the stability of the following compounds which are known in the prior art: 4-chloro-6,7-bis(2-methoxyethoxy)quinazoline (Comparative Example 14, compound A) and 4-(methylthio)-6,7-bis-(2-methoxyethoxy)quinazoline (Comparative Example 15, compound B).

The stability has been studied in solution, in particular in the solvent in which the conversion of the intermediates to erlotinib is carried out. In the case of the known compounds, the solvents are those described in the prior art aforementioned. Each experiment has been carried out with 100 mg of the compound to evaluate in 1 mL of solvent.

The results of the stability study of the individual intermediates in solution, at room temperature and 80°C are provided in Table 2. Purity measurements were performed by HPLC-MS.

**Table 2:**

| Compound | Purity To % | Storage | Solvent | Purity 1 day RT % | Purity 4 days RT % | Purity 1 day 80°C % | Purity 6 days 80°C % |
|---|---|---|---|---|---|---|---|
| Compound (II) Prepared as in Example 13 | 96.5 | 16 days RT | THF | 93.2 | 91.4 | 93.2 | |
| Compound A prepared according to Heterocycles 2007, vol. 71, pp.39-48 as mentioned in Comparative Example 14 | 88.9 | | DMF | 87.5 | 54.9 | 42.7 | |
| Compound A prepared according to Heterocycles 2007, vol. 71, pp.39-48 as mentioned in Comparative Example 14 | 97 | - | DMF | | | 89.5 | 11.2 |
| Compound B prepared according to Synth. Commun. 2007, vol. 37. pp.3409-3415 as mentioned in Comparative Example 15 | 86.2 | 2 days -20 °C | IPA | 86.4 | 84.0 | 87.8 | |

RT means room temperature; To refers to the moment of isolation of the given intermediate.

The previous results show that both the 4-(methylthio)-6,7-bis-(2-methoxyethoxy)quinazoline and the key intermediate of the preparation process of the present invention are stable.

However, the compound 4-chloro-6,7-bis(2-methoxyethoxy)quinazoline is not stable, in particular at 80 °C in solution of DMF, the solvent in which and the temperature at which the next reaction was carried out in the prior art. The stability at room temperature of compound A was also checked from a 4-chloro-6,7-bis(2-methoxyethoxy)quinazoline with a lower purity, obtained also according to Heterocycles 2007, vol. 71, pp.39-48. The results show that in solution of DMF at room temperature, compound A is also not stable.

In order to confirm the degradation that occurs due to the low stability under the reaction conditions of the starting material 4-chloro-6,7-bis(2-methoxyethoxy)quinazoline, erlotinib was prepared from this compound. Treatment of 4-chloro-6,7-bis(2-methoxyethoxy)quinazoline under the reaction conditions for 2 h prior to addition of the aniline reactant has a significant detrimental effect on the yield of erlotinib. The yield decreases from 90% to 74% and the amount of 6,7-bis(2-methoxyethoxy)quinazolinone (the hydrolysis product of 4-chloro-6,7-bis(2-methoxyethoxy)quinazoline) increases from 8.3% to 30.4% in the mother liquors.

### Example 18: Determination of the amount of the impurity described in WO2009/007984 in the erlotinib/erlotinib hydrochloride prepared by the process of the present invention.

In order to determine whether the erlotinib prepared by the process of the present invention contains the impurity mentioned in WO 2009/007984, namely N-[(3-ethynylphenyl)-2-methoxyethyl)]-6,7-bis(2-methoxyethoxy)-4-quinazolinamine (of which, as stated in this document, it is difficult to purify erlotinib, this impurity was prepared and was identified following the methods described in this patent application.

Thus, this impurity was prepared by alkylation of erlotinib. The alkylation was carried out in DMF at 80 °C for 24 h using bromoethyl methyl ether as alkylating agent. The reaction gave a mixture of starting material (60%) and three products (25%, 7.7% and 6.0%) having the same mass (m/z = 452). Based on the HPLC-MS results and on NMR spectroscopy, this could be a mixture of the three possible isomers, C, D and E, drawn below.

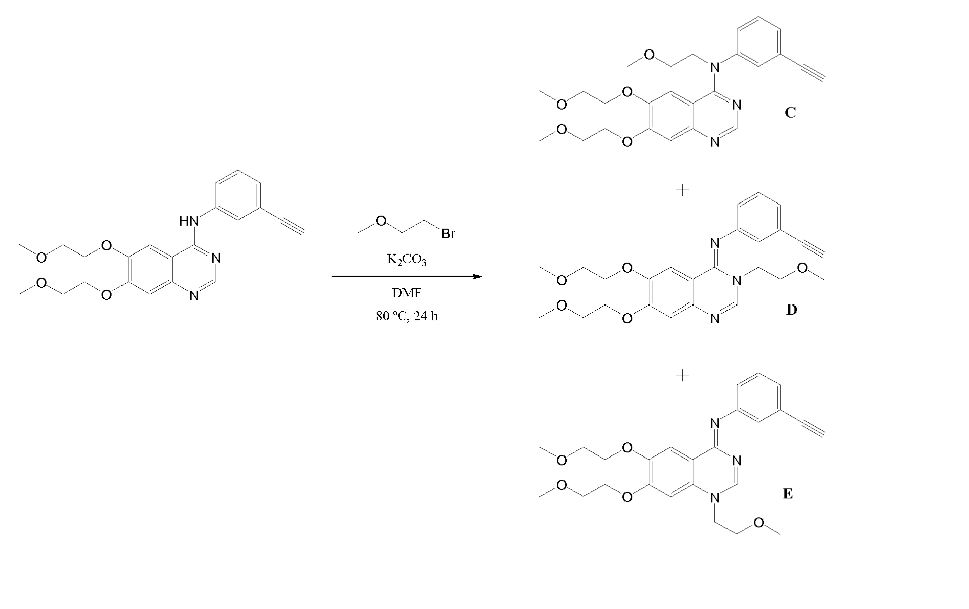

HPLC conditions: Column: XBridge C₁₈; eluent: NH₄HCO₃ pH 8/ACN (95:5-7 min-0:100); sample: 1 mg in 2 ml methanol; detection: 254nm.

Retention times are described in Table 3 (specific retention times for the structures drawn cannot be assigned with the information currently available).

**Table 3:**

| Product | Proportion % | Retention time (min) | Relative retention time (RRT) |
|---|---|---|---|
| 1 | 25.0 | 3.59 | 0.96 |
| Erlotinib | 60.1 | 3.75 | - |
| 2 | 7.7 | 3.92 | 1.05 |
| 3 | 6.0 | 4.25 | 1.13 |

The relative retention time described for impurity C of WO 2009/007984 is 1.14. None of these three regioisomeric products have been detected in the erlotinib / erlotinib hydrochloride obtained by the process of the present invention following the Examples included above.

### References cited in the description

- WO 96/30347
- Venkateshappa Chandregowda et al., Heterocycles 2007, vol. 71, pp. 39-48
- Petr Knesl et al., Molecules 2006, vol. 11, pp. 286-297
- WO 2009/007984
- Venkateshappa Chandregowda et al., Synthetic Communications 2007, vol.37, pp. 3409-3415
- Venkateshappa Chandregowda et al., Organic Process Research & Development 2007, vol . 11, pp. 813-816

## Claims

1. A preparation process of erlotinib of formula (I) or a salt thereof, 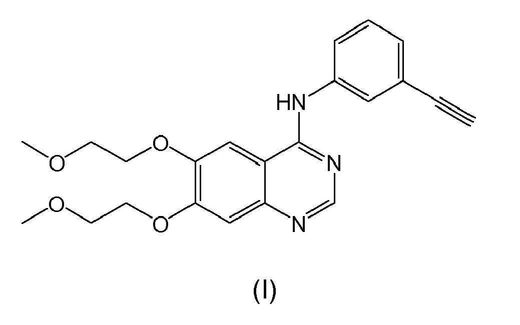 comprising:
(a)reacting a compound of formula (II) with a compound of formula (III) or a salt thereof, 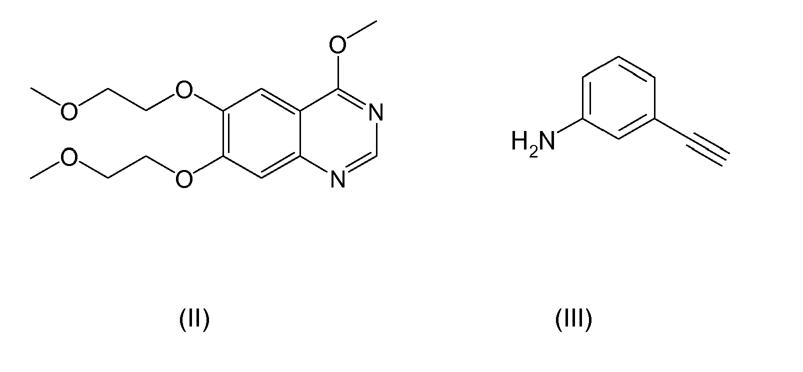 in the presence of a base and a reaction-inert solvent; and
(b) optionally, treating the compound obtained in step a) with a pharmaceutically acceptable acid to form the corresponding pharmaceutically acceptable salt.

2. The process according to claim 1, wherein the base is selected from the group consisting of: *n*-butyllithium, *sec*-butyllithium, hexyllithium, lithium diisopropylamide, *tert*-butyllithium, lithium hexamethyldisilazide, R₂NLi, and RLi, where R is a (C₁-C₈)-alkyl.

3. The process according to any of the claims 1-2, wherein the molar ratio of 3-ethynylaniline / base relative to compound (II) is comprised between 1.1 : 2.2 and 1.4 : 3.

4. The process according to any of the claims 1-3, wherein in a previous step a compound of formula (IV) is reacted with a compound of formula (V) to yield the compound of formula (II). 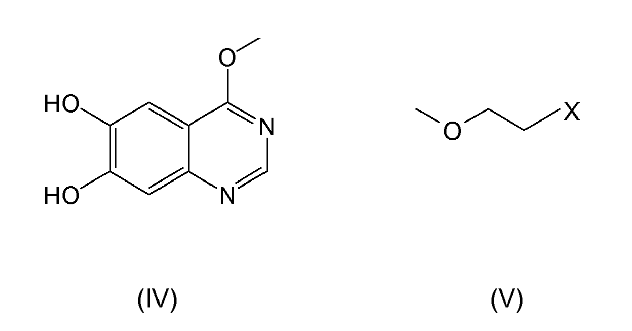 wherein X is a leaving group.

5. The process according to claim 4, wherein the leaving group is bromide.

6. The process according to any of the claims 4-5, wherein in a previous step carried out in one pot a compound of formula (VI) is reacted with a halogenating agent to yield a 4-halo-6,7-dihydroxyquinazoline and then with methanol to yield a compound of formula (IV).

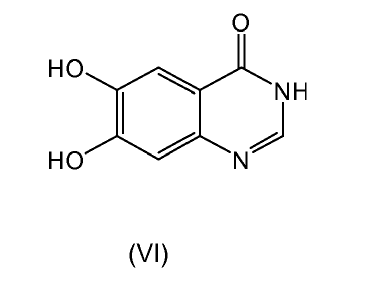

7. The process according to claim 6, wherein the halogenating agent is selected from the group consisting of SOCl₂, (COCl)₂, PBr₃, PBr₅, PCl₃, PCl₅, and POCl₃.

8. The process according to claim 7, wherein the 4-halo-6,7-dihydroxyquinazoline is 4-chloro-6,7-dihydroxyquinazoline and the halogenating agent is a chlorinating agent.

9. The process according to claim 8, wherein the halogenating agent is POCl₃.

10. The preparation process according to any of the claims 1-3, wherein in a previous step carried out in one pot a compound of formula (VII) is reacted with a halogenating agent to yield 4-halo-6,7-bis-(2-methoxyethoxy)-quinazoline, and then with methanol to yield a compound of formula (II).

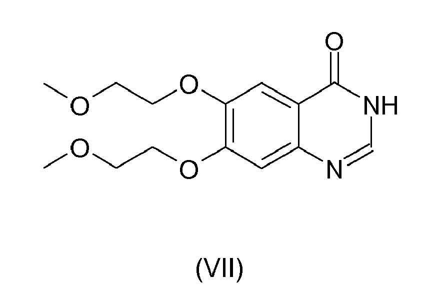

11. The process according to claim 10, wherein the halogenating agent is selected from the group consisting of SOCl₂, (COCl)₂,PBr₃, PBr₅, PCl₃, PCl₅, and POCl₃.

12. The process according to claim 11, wherein the 4-halo-6,7-bis-(2-methoxyethoxy)-quinazoline is 4-chloro-6,7-bis-(2-methoxyethoxy)-quinazoline and the halogenating agent is a chlorinating agent.

13. The process according to claim 12, wherein the halogenating agent is POCl₃.

14. A compound of formula (II) or a salt thereof.

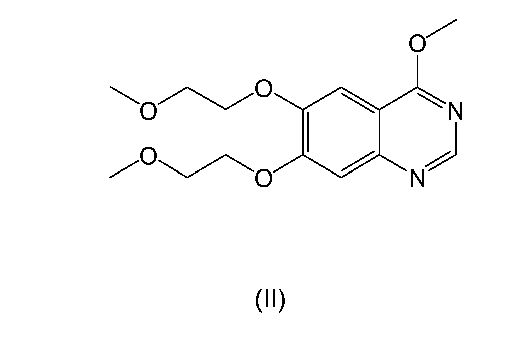

15. A compound of formula (IV) or a salt thereof.
